# FASCICULE DE BREVET EUROPEEN

(11) **EP 2 794 543 B1**
(45) Date de publication et mention de la délivrance du brevet: **25.05.2016**
(21) Numéro de dépôt: 12816761.6
(22) Date de dépôt: 21.12.2012
(51) Int. Cl.: C07C 51/285, C07C 53/126, C07C 55/08, C07C 55/12, C07C 55/18

(54) **PROCÉDÉ DE SCISSION MOLÉCULAIRE OXYDATIVE D'UN COMPOSÉ GRAS**
VERFAHREN ZUR OXIDATIVEN MOLEKULAREN SPALTUNG EINER FETTVERBINDUNG
METHOD OF OXIDATIVE MOLECULAR CLEAVAGE OF A FATTY COMPOUND

(30) Priorité: 22.12.2011 FR 1104028
(43) Date de publication de la demande: 29.10.2014
(73) Titulaire: Institut National Polytechnique de Toulouse (INPT), 31029 Toulouse Cedex 4 (FR); Institut National de la Recherche Agronomique (INRA), 75338 Paris Cedex 07 (FR)
(72) Inventeur: GODARD, Anaïs, F-31400 Toulouse (FR); THIEBAUD ROUX, Sophie, F-31240 L'union (FR); DE CARO, Pascale, F-31400 Toulouse (FR); VEDRENNE, Emeline, F-31130 Quint-Fonsegrives (FR); MOULOUNGUI, Zéphirin, F-31200 Toulouse (FR)
(74) Mandataire: Cabinet BARRE LAFORGUE & associés
(86) Numéro de dépôt international: PCT/FR2012/053037
(87) Numéro de publication internationale: WO 2013/093366

(56) Documents cités:
- JP-A- 5 004 938

## Description

L'invention concerne un procédé de préparation d'acides carboxyliques par scission moléculaire oxydative d'un composé gras. En particulier, l'invention concerne un procédé de préparation d'acides carboxyliques (notamment de monoacides carboxyliques, et/ou de diacides carboxyliques, et/ou d'acides carboxyliques ω-ester, et/ou d'acides carboxyliques hydroxylés) par scission moléculaire oxydative d'un composé gras choisi dans le groupe formé des acides carboxyliques aliphatiques insaturés, des esters -en particulier des esters d'alkyle, notamment des esters méthyliques- d'acide carboxylique aliphatique insaturé, des acides carboxyliques aliphatiques époxydés, des esters -en particulier des esters d'alkyle, notamment des esters méthyliques- d'acide carboxylique aliphatique époxydé, des acides carboxyliques aliphatiques insaturés hydroxylés et des esters -en particulier des esters d'alkyle, notamment des esters méthyliques- d'acide carboxylique aliphatique insaturé hydroxylé.

Un tel procédé de scission moléculaire oxydative d'un composé gras trouve ses applications dans le domaine de la fabrication d'acides gras à chaines courtes, notamment d'acides gras présentant une chaine principale de moins de 12 atomes de carbone, et en particulier d'acides gras présentant une chaine principale à nombre impair d'atomes de carbone.

En outre, ces composés -notamment l'acide azélaïque converti en particulier sous la forme d'esters- constitue un produit de départ pour la fabrication de nombreux produits industriels, notamment de polymères -tels que le nylon 6/9-, de plastifiants, d'adhésifs, de solvants, de lubrifiants biodégradables et d'inhibiteurs de corrosion. En outre, l'acide azélaïque constitue un principe actif de compositions cosmétiques comme composé kératolytique et comme antiacnéique. L'acide pélargonique est un intermédiaire de synthèse de différents lubrifiants, de plastifiants, de parfums, d'herbicides, de fongicides et de résines.

On connaît déjà des procédés permettant de réaliser une scission moléculaire oxydative d'acides gras insaturés. Ces procédés nécessitent l'utilisation de composés toxiques et/ou dangereux pour la santé humaine et/ou l'environnement, tels que l'acide chromique, l'acide nitrique, le permanganate de potassium, le tétroxyde d'osmium, les peracides, les périodates et les hypochlorites. On connaît aussi un procédé dans lequel on utilise de l'ozone, comme réactif oxydant, pour la scission moléculaire oxydative de l'acide oléique en acide pélargonique et en acide azélaïque.

De tels composés utilisés dans un procédé de scission moléculaire oxydative d'un composé gras ne sont pas respectueux de l'environnement.

En outre, le document (Pai et al., (2005), Russian Chemical Bulletin, International Edition, 54;8, 1847-1854) décrit un procédé de synthèse d'acide azélaïque et d'acide pélargonique par scission moléculaire oxydative catalytique d'acide oléique dans une solution de peroxyde d'hydrogène. Dans un tel procédé, on réalise d'abord la synthèse d'un catalyseur solide de formule générale [C₅H₅N(n-C₁₆H₃₃)]₃{PO₄[WO(O₂)₂]₄}. On place ensuite une quantité du catalyseur solide dans un réacteur. On ajoute ensuite l'acide oléique, puis on agite le mélange formé, puis on ajoute une solution de peroxyde d'hydrogène et on chauffe le mélange. On connait de JPH054938 un procédé de scission moléculaire oxydative d'acide oléique.

Dans un tel procédé, on réalise donc d'abord une étape spécifique de synthèse du catalyseur en l'absence d'acide oléique, puis on purifie ledit catalyseur avant de réaliser la scission moléculaire oxydative de l'acide oléique en acide azélaïque et en acide pélargonique. Un tel procédé ne permet pas de réaliser une synthèse de monoacide et de diacide carboxyliques en une seule étape. En particulier, un tel procédé nécessite la purification du catalyseur. Cette étape de purification entraîne des pertes de catalyseur solide lors de sa purification. Enfin, un tel procédé est complexe en cela qu'il nécessite une première étape de formation du catalyseur à réaliser à température ambiante, puis une seconde étape de scission oxydation de l'acide oléique à réaliser à la température de 80°C.

L'invention vise à pallier les inconvénients précédemment évoqués en proposant un procédé de scission moléculaire oxydative d'un composé gras qui ne nécessite pas l'utilisation d'un agent oxydant toxique. L'invention vise donc un procédé de scission moléculaire oxydative d'un composé gras qui est respectueux de l'environnement.

L'invention vise en particulier un tel procédé de scission moléculaire oxydative d'un composé gras qui présente un rendement amélioré par rapport aux procédés de l'état de la technique. En particulier, l'invention vise un tel procédé de scission moléculaire oxydative d'un composé gras, qui est réalisé en une seule étape et qui ne nécessite qu'un traitement simple de purification des produits de scission moléculaire oxydative dudit composé gras.

L'invention vise aussi un tel procédé de scission moléculaire oxydative d'un composé gras qui soit simple à mettre en oeuvre, qui ne nécessite pas de prévoir des installations spécifiques ou dangereuses. En particulier, l'invention vise un tel procédé de scission moléculaire oxydative d'un composé gras mis en oeuvre à pression atmosphérique.

L'invention vise ainsi un tel procédé de scission moléculaire oxydative d'un composé gras qui soit éco-compatible, c'est-à-dire qui ne nécessite pas pour sa mise en oeuvre de solvant organique qui soit toxique pour l'environnement et/ou pour la santé humaine ou animale et qui soit obtenu à partir de ressources fossiles -notamment du pétrole ou du gaz naturel- qui ne sont pas renouvelables.

L'invention vise ainsi un tel procédé de scission moléculaire oxydative d'un composé gras obtenu à partir d'une ressource respectueuse des préconisations européennes en termes de développement durable.

L'invention vise aussi un tel procédé de scission moléculaire oxydative d'un composé gras qui peut être produit à partir d'une ressource naturelle -notamment une ressource naturelle végétale issue de l'agriculture et/ou de la sylviculture- qui est renouvelable, telle que l'huile de tournesol qui est produite en grande quantité dans le sud de la France et dans les pays du pourtour méditerranéen.

L'invention vise un tel procédé de scission moléculaire oxydative d'un composé gras qui ne nécessite pas des étapes multiples de synthèse et de purification d'un catalyseur de scission moléculaire oxydative.

L'invention vise un tel procédé de scission moléculaire oxydative d'un composé gras qui permette de valoriser une ressource végétale qui est renouvelable, notamment l'huile de tournesol.

L'invention vise également à atteindre tous ces objectifs à moindre coût, en proposant un procédé de scission moléculaire oxydative d'un composé gras de faible coût de revient réalisée à partir de moyens, et notamment de composés organiques ou minéraux, usuels et peu onéreux.

L'invention vise également et plus particulièrement à proposer une telle solution qui soit compatible avec les contraintes de sécurité, de développement durable, de rentabilité et de respect de l'environnement.

Pour ce faire, l'invention concerne un procédé de scission moléculaire oxydative d'un composé gras choisi dans le groupe formé des acides carboxyliques aliphatiques insaturés, des esters d'un acide carboxylique aliphatique insaturé, des acides carboxyliques aliphatiques époxydés, des esters d'un acide carboxylique aliphatique époxydé, des acides carboxyliques aliphatiques insaturés hydroxylés et des esters d'un acide carboxylique aliphatique insaturé hydroxylé, dans lequel :
- on forme une composition, dite composition grasse, constituée d'au moins un acide carboxylique aliphatique, ladite composition grasse comprenant le composé gras ;
caractérisé en ce que :
- on ajoute ensuite à ladite composition grasse une solution d'au moins un sel d'ammonium quaternaire dans l'eau apte à former une émulsion dudit composé gras et de l'eau, puis ;
- on ajoute à ladite émulsion une solution liquide d'au moins un acide tungsto-phosphorique dans une composition comprenant du peroxyde d'hydrogène (H₂O₂) ;
de façon à *former in situ* dans l'émulsion une quantité d'un catalyseur, dit catalyseur de transfert de phase, formé de l'acide tungsto-phosphorique et d'au moins un ammonium quaternaire du (des) sel(s) d'ammonium quaternaire(s) et à permettre la scission moléculaire oxydative du composé gras.

L'invention consiste donc à proposer un procédé de scission moléculaire oxydative d'un composé gras dans lequel on réalise une émulsion du composé gras et d'une solution aqueuse d'au moins un sel ammonium quaternaire puis on ajoute à ladite émulsion au moins un acide tungsto-phosphorique de formule H₃PW₁₂O₄₀ et du peroxyde d'hydrogène de façon à former *in situ* dans ladite émulsion un catalyseur, dit catalyseur de transfert de phase, apte à se distribuer dans l'émulsion et à permettre une scission moléculaire oxydative du composé gras et la formation de monoacides carboxyliques, et/ou de diacides carboxyliques, et/ou d'acides carboxyliques ω-ester, et/ou d'acides carboxyliques hydroxylés. Un tel procédé ne nécessite donc aucune étape de purification dudit catalyseur de transfert de phase.

En effet, les inventeurs ont constaté qu'un tel procédé de scission moléculaire oxydative d'un composé gras, dans lequel on réalise la synthèse d'un catalyseur de transfert de phase *in situ* dans le milieu de réaction formé d'une émulsion dudit composé gras et d'une phase aqueuse permet d'augmenter le rendement de ladite réaction d'oxydation au-delà du rendement de scission moléculaire oxydative obtenu dans une réaction comparable de scission moléculaire oxydative réalisée avec une quantité comparable de catalyseur de transfert de phase préalablement préparé avec la même quantité de sel d'ammonium quaternaire et d'acide tungsto-phosphorique.

Les inventeurs supposent qu'un procédé de scission moléculaire oxydative d'un composé gras selon l'invention permet une dispersion améliorée du catalyseur de transfert de phase dans l'émulsion du composé gras et de l'eau.

Avantageusement, la composition grasse est une composition liquide.

Avantageusement, chaque acide tungsto-phosphorique est un acide tungsto-phosphorique hydraté de formule générale (I) suivante :

H₃PW₁₂O₄₀, nH₂O (I),

dans laquelle n est un nombre décimal.

Avantageusement, l'acide tungsto-phosphorique est adapté pour former *in situ* dans l'émulsion une quantité d'un catalyseur de transfert de phase formé de l'acide tungsto-phosphorique sous forme péroxo (comprenant au moins une liaison -O-O-) et de chaque ammonium quaternaire.

Avantageusement, on ajoute ensuite à ladite composition grasse une solution d'au moins un sel d'ammonium quaternaire dans l'eau apte à former une émulsion dudit composé gras et de l'eau.

Avantageusement, la composition grasse comprend au moins un acide carboxylique choisi dans le groupe formé des acides carboxyliques aliphatiques mono-insaturés (par exemple l'acide oléique, l'acide palmitoléique) et des acides carboxyliques aliphatiques poly-insaturés (par exemple l'acide linoléique, l'acide arachidonique).

Avantageusement, dans une première variante d'un procédé selon l'invention, la composition grasse est constituée majoritairement du composé gras choisi dans le groupe formé des acides carboxyliques aliphatiques insaturés -notamment de l'acide oléique et de l'acide linoléique- et des acides carboxyliques aliphatiques époxydés -notamment de l'acide 9,10-époxyoctadécanoïque-, des esters d'acides carboxyliques aliphatiques -notamment l'oléate de méthyle- et des acides carboxyliques hydroxylés -notamment de l'acide ricinoléique-.

Avantageusement, dans une deuxième variante d'un procédé selon l'invention, la composition grasse est constituée essentiellement (à l'exception de traces éventuelles d'acides gras saturés ou insaturés résiduels) du composé gras choisi dans le groupe formé des acides carboxyliques aliphatiques insaturés -notamment de l'acide oléique et de l'acide linoléique- et des acides carboxyliques aliphatiques époxydés -notamment de l'acide 9,10-époxyoctadécanoïque- et des esters d'acides carboxyliques aliphatiques -notamment l'oléate de méthyle-.

Avantageusement, dans une troisième variante d'un procédé selon l'invention, la composition grasse est un mélange comprenant au moins un composé gras choisi dans le groupe formé des acides carboxyliques aliphatiques insaturés, des esters d'un acide carboxylique aliphatique insaturé, des acides carboxyliques aliphatiques époxydés, des esters d'un acide carboxylique aliphatique époxydé, des acides carboxyliques aliphatiques insaturés hydroxylés -notamment choisi dans le groupe formé des acides carboxyliques aliphatiques insaturés α-hydroxylés et des acides carboxyliques aliphatiques insaturés β-hydroxylés-, des esters d'un acide carboxylique aliphatique insaturé hydroxylé et au moins un acide ou un ester d'acide gras saturé -notamment un acide palmitique, un palmitate d'alkyle (en particulier un palmitate de méthyle), un acide stéarique, un stéarate d'alkyle (en particulier un stéarate de méthyle), un acide myristique, d'un myristate d'alkyle (en particulier un myristate de méthyle)-. En général, la composition grasse comprend au moins un composé gras choisi dans le groupe formé des acides carboxyliques aliphatiques insaturés et de leurs esters d'alkyle, des acides carboxyliques aliphatiques époxydés et de leurs esters d'alkyle et des acides carboxyliques aliphatiques insaturés hydroxylés -notamment choisi dans le groupe formé des acides carboxyliques aliphatiques insaturés α-hydroxylés et des acides carboxyliques aliphatiques insaturés β-hydroxylés- et de leurs esters d'alkyle et comprend aussi une fraction molaire d'au moins un acide carboxylique aliphatique issu de l'hydrolyse -enzymatique ou chimique- d'au moins un triglycéride. Avantageusement, on obtient la composition grasse par hydrolyse enzymatique de l'huile de tournesol. A titre d'exemple, le profil en acides gras d'une telle composition grasse obtenue par hydrolyse enzymatique de l'huile de tournesol peut comprendre une proportion massique de l'ordre de 87 % d'acide oléique, une proportion massique de l'ordre de 5 % d'acide linoléique, une proportion massique de l'ordre de 3 % d'acide palmitique, une proportion massique de l'ordre de 3 % d'acide stéarique, une proportion massique de l'ordre de 0,2 % d'acide caprique. De façon générale, une telle composition grasse comprend au moins un composé gras choisi dans le groupe formé des acides carboxyliques aliphatiques insaturés et des acides carboxyliques aliphatiques époxydés et au moins un acide gras choisi parmi les acides gras saturés (acide laurique, acide myristique, acide palmitique, acide stéarique) et les acides gras polyinsaturés (acide oléique, acide linoléique, acide linolénique, acide arachidonique).

Avantageusement et selon l'invention, on choisit le composé gras dans le groupe formé des composés gras de formule générale (II) suivante :

R₁-(CH₂)ₐ-R₂-(CH₂)_{b}-R₃-(CH₂)_{c}-R₄-(CH₂)_{d}-R₅-(CH₂)ₑ-R₆ (II),

dans laquelle :
o R₁ et R₆ sont deux groupements d'atomes identiques ou distincts choisis parmi un méthyle (-CH₃), un carboxyle (-COOH) et un groupement ester de formule générale -COOR₇ dans laquelle R₇ est un groupement hydrocarboné aliphatique linéaire comprenant de 1 à 8 atomes de carbone ;
o R₂, R₃, R₄ et R₅ sont des groupements d'atomes identiques ou distincts choisis parmi un groupe -CH₂- un groupe époxyde 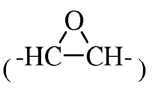 et un groupe (-CH=CH-) ;
o a, b, c, d et e sont des nombres entiers naturels identiques ou distincts de l'intervalle [0 ; 15].

Avantageusement, on choisit le composé gras dans le groupe formé de l'acide oléique, de l'acide linoléique, de l'acide linolénique, de l'acide arachidonique, de l'acide palmitoléique (ou acide 9-cis-hexadécènoïque), de l'acide érucique (acide docos-13-énoïque), de l'acide brassidique (acide trans-13-dococénoïque), de l'acide ricinoléique et de l'acide 9-époxyoctadécanoïque.

Avantageusement et selon l'invention, on choisit chaque sel d'ammonium quaternaire dans le groupe formé des sels d'ammonium quaternaire de formule générale (III) suivante :

A₁A₂A₃A₄N⁺X⁻ (III) ;

dans laquelle :
o A₁, A₂, A₃ et A₄ sont des groupements hydrocarbonés aliphatiques identiques ou distincts présentant un nombre d'atomes de carbone inférieur à 10, et ;
o X⁻ est un anion -notamment choisi dans le groupe formé d'un chlorure, d'un nitrate et d'un bromure-.

Avantageusement, l'ammonium quaternaire est choisi dans le groupe formé des alkyl-pyridinium. Avantageusement, l'ammonium quaternaire est l'hexadécyl-pyridinium de formule suivante :

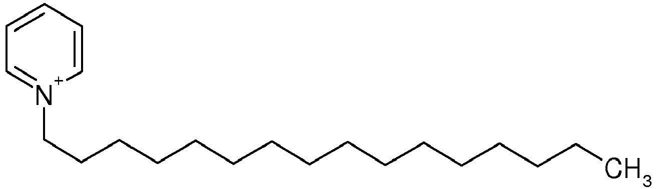

Avantageusement, l'ammonium quaternaire est choisi dans le groupe formé du *N*,*N*,*N*,*N-*tétrabutyl-ammonium, du *N,N,N*-tributyl*-N-*méthyl*-* ammonium et du *N*,*N*,*N*,*N*-tétrahexyl-ammonium.

Avantageusement et selon l'invention, la composition grasse est exempte de solvant organique. On réalise la scission moléculaire oxydative du composé gras dans l'émulsion formée du composé gras et de l'eau en présence du catalyseur de transfert de phase formé *in situ* dans l'émulsion et sans addition d'aucun solvant organique -notamment d'un solvant organique volatile- choisi dans le groupe formé du dichlorométhane (CH₂Cl₂), du chloroforme (CHCl₃), de l'éther éthylique ((H₃C-CH₂)₂O), du *tertio*-butanol (^{t}BuOH), et de l'acétonitrile (CH₃CN).

Avantageusement et selon l'invention, le composé gras est l'acide oléique (acide (9*cis*)-octadéc-9-énoïque) et les produits formés par scission moléculaire oxydative de l'acide oléique sont l'acide azélaïque (HOOC-(CH₂)₇-COOH) et l'acide pélargonique (H₃C-(CH₂)₇-COOH).

Avantageusement et selon l'invention, le composé gras est l'acide linoléique (acide (9*cis*, 12*cis*)-octadéca-9,12-diènoïque) et les produits formés par scission moléculaire oxydative de l'acide linoléique sont l'acide azélaïque (acide nonane-1,9-dioïque, HOOC-(CH₂)₇-COOH), l'acide malonique (acide 1,3-propanedioïque, HOOC-CH₂-COOH) et l'acide hexanoïque (H₃C-(CH₂)₄-COOH).

Avantageusement et selon l'invention, le composé gras est l'acide 9,10-époxyoctadécanoïque et les produits formés par scission moléculaire oxydative de l'acide 9,10-époxyoctadécanoïque sont l'acide azélaïque (acide nonane-1,9-dioïque, HOOC-(CH₂)₇-COOH) et l'acide pélargonique (H₃C-(CH₂)₇-COOH).

Avantageusement et selon l'invention, le composé gras est l'acide arachidonique et les produits formés par scission moléculaire oxydative de l'acide arachidonique sont l'acide hexanoïque (H₃C-(CH₂)₄-COOH), l'acide malonique (HOOC-CH₂-COOH) et l'acide pentanedioïque (HOOC-(CH₂)₃-COOH).

Avantageusement et selon l'invention, le composé gras est l'acide palmitoléique et les produits formés par scission moléculaire de l'acide palmitoléique sont l'acide azélaïque (HOOC-(CH₂)₇-COOH) et l'acide heptanoïque (H₃C-(CH₂)₅-COOH).

Avantageusement et selon l'invention, le composé gras est l'acide érucique (ou l'acide docos-13-énoïque) et les produits formés par scission moléculaire de l'acide palmitoléique sont l'acide pélargonique (H₃C-(CH₂)₇-COOH) et l'acide 1,13-tridécanedioïque (HOOC-(CH₂)₁₁-COOH).

Avantageusement et selon l'invention, le composé gras est l'acide linolénique (acide octadéca-triènoïque) et les produits formés par scission moléculaire oxydative de l'acide linolénique sont l'acide azélaïque (acide nonane-1,9-dioïque, HOOC-(CH₂)₇-COOH), l'acide propionique (H₃C-CH₂-COOH) et l'acide malonique (HOOC-CH₂-COOH).

Avantageusement et selon l'invention, le composé gras est l'acide ricinoléique (acide (9*cis*)-12-hydroxyoctadéca-9-ènoïque) et les produits formés par scission moléculaire oxydative de l'acide ricinoléique sont l'acide azélaïque (acide nonane-1,9-dioïque, HOOC-(CH₂)₇-COOH), l'acide 3-hydroxynonanoïque (HOOC-CH₂-CHOH-(CH₂)₅-CH₃).

Les inventeurs ont observé que le traitement d'un acide carboxylique insaturé hydroxylé par un procédé de scission oxydative selon l'invention conduit à l'oxydation de la double liaison, mais préserve le groupement hydroxyle.

Avantageusement et selon l'invention, la proportion molaire des insaturations (nombre d'équivalent molaire de double liaisons et/ou cycle époxyde du composé gras dans l'émulsion) du composé gras et du peroxyde d'hydrogène introduits dans l'émulsion est comprise entre 1/4 et 1/10 -notamment de l'ordre de 1/5-.

Avantageusement et selon l'invention, la proportion molaire des insaturations (nombre d'équivalent molaire de double liaisons et/ou cycle époxyde du composé gras dans l'émulsion) du composé gras et de l'acide tungsto-phosphorique introduits dans l'émulsion est comprise entre 1 % et 5 %.

Avantageusement et selon l'invention, l'acide tungsto-phosphorique et l'ammonium quaternaire introduits dans l'émulsion présentent une proportion équimolaire dans l'émulsion.

Avantageusement et selon l'invention, on réalise la scission moléculaire oxydative du composé gras à une température comprise entre 60°C et 120°C -notamment entre 70°C et 100°C, de préférence entre 80°C et 90°C-.

Avantageusement et selon l'invention, à l'issue de la scission moléculaire oxydative du composé gras, on récupère par filtration -à froid à une température inférieure à +4°C- le catalyseur de transfert de phase et on utilise ledit catalyseur dans une étape subséquente de scission moléculaire oxydative d'un second composé gras. Avantageusement, le second composé gras est identique ou distinct du composé gras.

L'invention concerne également un procédé caractérisé en combinaison par tout ou partie des caractéristiques mentionnées ci-dessus ou ci-après.

D'autres buts, caractéristiques et avantages de l'invention apparaîtront à la lecture des exemples suivants donnés uniquement à titre de description non limitative.

### EXEMPLE I - préparation in situ du catalyseur de transfert de phase.

Dans un procédé de scission moléculaire oxydative d'un composé gras et de préparation d'acides carboxyliques (notamment d'acide azélaïque (COOH-(CH₂)₇-COOH) et d'acide pélargonique (CH₃-(CH₂)₇-COOH)) selon l'invention, on prépare d'abord une composition d'acide gras contenant de l'acide oléique à partir d'une huile de tournesol à forte teneur en acide oléique (ARTERRIS, Toulouse, France). On réalise une hydrolyse enzymatique de cette huile de tournesol lors de laquelle on place 22,5 Kg d'huile de tournesol en contact avec une solution d'une lipase (Lyven, Colombelles, France) de *Candida cylindracea* dans de l'eau distillée (20,1 Kg) sous agitation magnétique à 40°C pendant 5 heures. On forme une préparation d'acides gras dont la composition, déterminée par chromatographie en phase gazeuse est donnée dans le tableau 1 ci-après.

**Tableau 1**

| Acide gras | Composition massique, % |
|---|---|
| Acide oléique, C_{18 :1} | 87,6 |
| Acide linoléique, C_{18:2} | 4,7 |
| Acide palmitique, C_{16:0} | 3,5 |
| Acide stéarique, C_{18:0} | 3,1 |
| Acide caprique, C_{10 :0} | 0,2 |
| Autres | 0,9 |

On place 21 g de cette préparation d'acides gras comprenant 65 mmole d'acide oléique dans un ballon tricol à fond rond de 250 mL équipé d'un réfrigérant, d'un agitateur mécanique et d'un dispositif de chauffage. On ajoute goutte à goutte 2 mL d'une solution aqueuse d'un sel d'ammonium quaternaire (3,36 mmole) choisi dans le groupe formé du chlorure de tétrabutyl-ammonium (*n*-Bu₄NCℓ, Sigma Aldrich, Saint-Quentin Fallavier, France), du bromure de tétrabutyl-ammonium (*n*-Bu₄NBr, Sigma Aldrich, Saint-Quentin Fallavier, France), du chlorure de *N*-cétylpyridinium monohydrate (C₅H₅N(n-C₁₆H₃₃)₃Cl, H₂O, Sigma Aldrich, Saint-Quentin Fallavier, France), du chlorure de *N*-méthyl-*N*,*N*,*N*-trioctyl-ammonium (CH₃N(n-C₈H₁₇)₃Cℓ, Sigma Aldrich, Saint-Quentin Fallavier, France) plus connu sous la dénomination « aliquat 336 » et du chlorure de *N,N,N,N-*tétraoctyl-ammonium (N(n-C₈H₁₇)₄Cℓ, Sigma Aldrich, Saint-Quentin Fallavier, France). On forme par agitation mécanique du mélange obtenu une émulsion de l'acide oléique et de la solution du sel d'ammonium quaternaire.

On prépare par mélange et agitation à température ambiante pendant 30 min, 4 g (1,2 mmole) d'acide tungsto-phosphorique (H₃PW₁₂O₄₀•15,4 H₂O) et 34 mL d'eau oxygénée à 30 % (325,0 mmole) dans 5 mL d'eau distillée. On ajoute la solution d'acide tungsto-phosphorique à l'émulsion de l'acide oléique et du sel d'ammonium quaternaire. On réalise l'addition de la solution d'acide tungsto-phosphorique goutte à goutte dans le ballon tricol contenant l'émulsion pendant une durée de 5 min. Après addition de la solution d'acide tungsto-phosphorique dans l'émulsion, on chauffe le milieu réactionnel à la température de 60°C. On place et on maintient le réacteur sous agitation mécanique (400 rpm) à la température de 85°C et à pression atmosphérique pendant une durée de 5 heures, puis on laisse le réacteur refroidir jusqu'à atteindre la température ambiante. On ajuste le pH du milieu de réaction à la valeur de pH = 1 par addition d'un volume d'une solution aqueuse d'acide chlorhydrique à la concentration de 4 mole/L.

Aux fins de la séparation des acides carboxyliques formés et du catalyseur, un volume d'acétate d'éthyle est ajouté au mélange acide à pH = 1 puis le milieu réactionnel est placé à une température (T_{précip}, température ambiante ou température inférieure à 4°C) de précipitation de façon à former un précipité du catalyseur. Le précipité formé est lavé à l'acétate d'éthyle. La phase aqueuse contenant les sels est séparée de la phase organique dans une ampoule à décanter puis est lavée à l'acétate d'éthyle. Les différentes phases organiques sont regroupées, séchées sur sulfate d'ammonium (Na₂SO₄) et évaporées sous pression réduite.

Les échantillons obtenus sont analysés et quantifiés par chromatographie en phase gazeuse au moyen d'un chromatographe Varian couplé à un détecteur à ionisation de flamme (FID) et équipé d'une colonne capillaire (L 50 m, Ø 0,25 mm, granulométrie 25 µm) pour l'analyse des esters méthyliques d'acides gras. La phase mobile est de l'hélium (Air liquide, France) à une pression de 1034 hPa (15 psi) en tête de colonne capillaire. La température de l'injecteur et du détecteur est de 250°C. La température du four renfermant la colonne est maintenue à 100°C pendant 5 min puis est accrue progressivement jusqu'à 180°C à une vitesse de 5°C/min pendant 10 min et finalement accrue progressivement jusqu'à 250°C à une vitesse de 10°C/min pendant 5 min et maintenue à cette température pendant 43 min.

Aux fins d'analyse, on réalise une solution de chaque échantillon à une concentration de 10 mg/mL dans le méthyl-*tertio*butyl-éther (MTBE). Les acides gras sont convertis en esters méthyliques par traitement à l'hydroxyde de tri-méthyl-sulfonium. On ajoute à titre d'étalon interne de l'acide pentadécanoïque à une concentration de 2 mg/mL. Les résultats sont présentés au tableau 2 ci-après, dans lequel T_{précip} est la température de précipitation, AZA % et PEA % représentent respectivement la valeur du rendement de synthèse et d'extraction de l'acide azélaïque et de l'acide pélargonique par rapport à l'acide oléique de départ.

**Tableau 2**

| Catalyseur préparé *in situ* | T_{précip} | AZA, % | PEA, % |
|---|---|---|---|
| (*n*-Bu₄N)₃{PO₄[WO(O₂)₂]₄} ; (A) | 4°C | 77,6 | 80,9 |
| (*n*-Bu₄N)₃{PO₄[WO(O₂)₂]₄} ; (A) | RT | 72,9 | 73,9 |
| (C₅H₅N(*n*-C₁₆H₃₃))₃{PO₄[WO(O₂)₂]₄}; (B) | 4°C | 81,5 | 86,1 |
| (C₅H₅N(*n*-C₁₆H₃₃))₃{PO₄[WO(O₂)₂]₄}; (B) | RT | 71,8 | 70,9 |
| (CH₃N(*n*-C₅H₁₇)₃)₃{PO₄[WO(O₂)₂]₄}; (C) | 4°C | 78,5 | 82,0 |
| (CH₃N(*n*-C₈H₁₇)₃)₃{PO₄[WO(O₂)₂]₄}; (C) | RT | 71,3 | 77,2 |
| ((*n*-C₈H₁₇)₄N)₃{PO₄[WO(O₂)₂]₄}; (D) | 4°C | 73,2 | 76,5 |

On observe un rendement amélioré pour une précipitation du catalyseur réalisée à la température de +4°C en comparaison avec une précipitation du catalyseur réalisée à la température ambiante.

### EXEMPLE 2 - Essai comparatif - Préparation préalable du catalyseur de transfert de phase.

On prépare préalablement les catalyseurs (A) et (B) décrits à l'exemple 1 par un procédé connu en lui-même de l'homme du métier. En particulier, un tel procédé est décrit dans (Pai et al., (2005), Russian Chemical Bulletin, 54 ; 8, 1847-1854). Dans un tel procédé, on ajoute une solution de peroxyde d'hydrogène (H₂O₂) à 30 % (34 mL, 325 mmole) à une solution d'acide tungsto-phosphorique (H₃PW₁₂O₄₀•15,4 H₂O, 4 g, 1,2 mmole) et on maintient sous agitation ce mélange pendant 30 minutes. On ajoute une solution aqueuse de 2 mL d'un sel d'ammonium quaternaire (3,36 mmole) choisi dans le groupe formé du chlorure de tétrabutyl-ammonium (n-Bu₄NCl, Sigma Aldrich, Saint-Quentin Fallavier, France) et du chlorure de *N*-cétylpyridinium monohydraté (C₅H₅N(n-C₁₆H₃₃)₃Cℓ, H₂O, Sigma Aldrich, Saint-Quentin Fallavier, France). On filtre et on lave à l'eau le précipité solide ainsi formé.

On mélange une quantité du catalyseur formé ci-dessus et de l'acide oléique (21 g, 65 mmole) dans un ballon tri-col à fond rond de 250 mL équipé d'un réfrigérant, d'un agitateur mécanique et d'un dispositif de chauffage. On chauffe le mélange à 60°C et on ajoute goutte à goutte pendant 5 min une solution de peroxyde d'hydrogène (H₂O₂, 325 mmole). Le mélange réactionnel est chauffé à 85°C et maintenu à la pression atmosphérique pendant 5 heures sous agitation magnétique (400 rpm). On laisse le réacteur refroidir jusqu'à atteindre la température ambiante. Le milieu réactionnel est ensuite traité et analysé comme à l'exemple 1. Les résultats des analyses sont donnés à titre de comparaison au tableau 3 ci-après.

**Tableau 3**

| Catalyseur | T_{précip} | AZA, % | PEA, % |
|---|---|---|---|
| (*n*-Bu₄N)₃{PO_{4[}WO(O₂)₂]₄} ; (A) | 4°C | 52,0 | 57,1 |
| (C₅H₅N(*n*-C₁₆H₃₃))₃{PO₄[WO(O₂)₂]₄}; (B) | 4°C | 70,2 | 75,1 |

On observe un rendement de la réaction de conversion de l'acide oléique en acide azélaïque et en acide pélargonique qui est amélioré pour la synthèse réalisée par formation *in situ* du catalyseur (tableau 2).

Avec le catalyseur (A) et un traitement à 4°C, le rendement de la réaction passe d'une valeur de 52 % (acide azélaïque) et 57,1 % (acide pélargonique) pour une préparation préalable du catalyseur (tableau 3) à une valeur de 77,6 % (acide azélaïque) et 80,9 % (acide pélargonique) pour une synthèse *in situ* du catalyseur (tableau 2) soit une augmentation supérieure à 40 %. Avec le catalyseur (B) et un traitement à 4°C, le rendement de la réaction passe d'une valeur de 70,2 % (acide azélaïque) et 75,1 % (acide pélargonique) pour une préparation préalable du catalyseur (tableau 3) à une valeur de 81,5 % (acide azélaïque) et 86,1 % (acide pélargonique) pour une synthèse *in situ* du catalyseur (tableau 2) soit une augmentation de l'ordre de 15 %.

### EXEMPLE 3 - Choix du sel d'ammonium quaternaire

On réalise un traitement de scission moléculaire oxydative de l'acide oléique tel que décrit à l'exemple 1 dans lequel les proportions molaires des réactifs sont :
- acide oléique : 1 équivalent ;
- H₂O₂ : 5 équivalents ;
- catalyseur formé *in situ :* 0,02 équivalent.

Le mélange réactionnel est chauffé à 85°C et maintenu à la pression atmosphérique pendant 5 heures sous agitation mécanique (400 rpm). Pour la préparation *in situ* du catalyseur, on choisit le sel d'ammonium quaternaire décrit au tableau 4 ci-après. La colonne « OLA % » représente la fraction molaire d'acide oléique transformée lors de la réaction.

**Tableau 4**

| Sel d'ammonium | X⁻ | OLA, % | AZA, % | PEA, % |
|---|---|---|---|---|
| Aucun | - | 38,1 | 1,8 | 2,2 |
| (*n-*Bu₄N)⁺X*⁻* | Cℓ⁻ | 100 | 77,6 | 80,9 |
| (*n*-Bu,N)⁺X⁻ | Br | 100 | 71,8 | 76,2 |
| C₅H₅N(*n*-C₁₆H₃₃)⁺X⁻ | Cℓ⁻ | 100 | 81,5 | 86,1 |
| CH₃N(*n*-C₈H₁₇)₃⁺X⁻ | Cℓ⁻ | 100 | 75,7 | 80,7 |
| N(*n*-C₈H₁₇)₄⁺X⁻ | Cℓ⁻ | 100 | 73,2 | 76,5 |

En l'absence de catalyseur, 38,1 % de l'acide oléique de départ disparait et permet la formation de nombreux produits dont seulement 1,8 % d'acide azélaïque et 2,2 % d'acide pélargonique.

La formation *in situ* de catalyseurs à partir des sels d'ammonium (*n*-Bu₄,N)⁺Cℓ⁻, (*n*-Bu₄,N)⁺Br⁻, C₅H₅N(*n*-C₁₆H₃₃)⁺Cℓ⁻, CH₃N(*n*-C₈H₁₇)₃⁺Cℓ⁻ et N(*n*-C₈H₁₇)₄⁺Cℓ⁻ permet de transformer 100 % de l'acide oléique de départ et de former de l'acide azélaïque et de l'acide pélargonique avec un rendement supérieur à 73 % pour l'acide azélaïque et supérieur à 76 % pour l'acide pélargonique.

### EXEMPLE 4 - Effet de la température

On réalise un traitement de scission moléculaire oxydative de l'acide oléique tel que décrit à l'exemple 1 dans lequel on forme *in situ* l'un des catalyseurs (A), (B), (C) ou (D) et on réalise la synthèse à une température T_{réaction} précisée au tableau 5 ci-après et dans lequel la valeur « OLA % » représente la fraction molaire d'acide oléique transformée lors de la réaction, « AZA % » et « PEA % » représentent respectivement la valeur du rendement de synthèse et d'extraction de l'acide azélaïque et de l'acide pélargonique par rapport à l'acide oléique de départ.

**Tableau 5**

| Catalyseur préparé *in situ* | T_{réaction}, °C | OLA % | AZA % | PEA % |
|---|---|---|---|---|
| (*n*-Bu₄N)₃{PO₄[WO(O₂)₂]₄} ; (A) | 75 | 100 | 59,2 | 66,5 |
| (A) | 85 | 100 | 77,6 | 80,9 |
| (A) | 95 | 99,5 | 65,8 | 73,3 |
| (C₅H₅N(*n*-C₁₆H₃₃))₃{PO₄[WO(O₂)₂]₄} ; (B) | 65 | 96,6 | 40,8 | 44,2 |
| (B) | 75 | 100 | 66,8 | 71,1 |
| (B) | 85 | 100 | 81,5 | 86,1 |
| (B) | 95 | 100 | 77,6 | 80,2 |
| (B) | 105 | 99,3 | 60,9 | 60,5 |
| (CH₃N(*n*-C8H₁₇)₃)₃{PO₄[WO(O₂)₂]₄}; (C) | 75 | 100 | 69,2 | 74,8 |
| (C) | 85 | 100 | 75,7 | 80,7 |
| (C) | 95 | 99,8 | 78,5 | 82,0 |
| (C) | 105 | 100 | 71,5 | 76,1 |
| (C) | 115 | 100 | 72,8 | 77,2 |

Les meilleurs rendements sont obtenus à une température de l'ordre de 85°C pour les catalyseurs (A) et (B) et de l'ordre de 95°C pour le catalyseur (C).

### EXEMPLE 5 - Proportion de catalyseur

On réalise un traitement de scission moléculaire oxydative de l'acide oléique tel que décrit à l'exemple 1 dans lequel on forme *in situ* le catalyseur (B) dans une proportion molaire précisée au tableau 6 ci-après et dans lequel la valeur « OLA % » représente la fraction molaire d'acide oléique transformée lors de la réaction, « AZA % » et « PEA % » représentent respectivement la valeur du rendement de synthèse de l'acide azélaïque et de l'acide pélargonique par rapport à l'acide oléique de départ. La quantité d'acide oléique (OLA) initiale est de 65 mmoles et la quantité de H₂O₂ initiale est de 325 mmoles (30 %). Le rapport molaire OLA/H₂O₂ initial est de 1/5, la température est de 85°C, la durée de réaction est de 5 heures et la vitesse d'agitation est de 400 rpm.

**Tableau 6**

| Catalyseur (B) préparé *in situ* (C₅H₅N(*n*-C₁₆H₃₃))₃{PO₄[WO(O₂)₂]₄} mole % par rapport à OLA | OLA % | AZA % | PEA % |
|---|---|---|---|
| 1 | 100 | 69,3 | 68,3 |
| 2 | 100 | 81,5 | 86,1 |
| 4 | 100 | 70,9 | 62,3 |

La proportion molaire de catalyseur formé *in situ* permettant d'obtenir le meilleur rendement est de l'ordre de 2 %.

### EXEMPLE 6 - Vitesse d'agitation

On réalise un traitement de scission moléculaire oxydative de l'acide oléique tel que décrit à l'exemple 1 dans lequel on forme *in situ* le catalyseur (B) dans une proportion molaire de 2 %. La quantité d'acide oléique (OLA) initiale est de 65 mmoles et la quantité de H₂O₂ initiale est de 325 mmoles (30 %). Le rapport molaire OLA/H₂O₂ initial est de 1/5, la température est de 85°C, la durée de réaction est de 5 heures et la vitesse de rotation de l'agitateur mécanique est comprise entre 250 rpm et 1250 rpm.

On observe une diminution du rendement de synthèse de l'acide azélaïque et de l'acide pélargonique pour une vitesse de rotation de l'agitateur mécanique supérieure à 800 rpm. Les inventeurs supposent qu'une vitesse de rotation de l'agitateur mécanique supérieure à 800 rpm est de nature à permettre l'introduction de bulles d'air par cavitation dans le milieu réactionnel et la diminution de la surface de contact entre le catalyseur et le substrat.

En outre, une diminution du rendement est aussi observée pour une vitesse de rotation de l'agitateur mécanique inférieure à 300 rpm. Les inventeurs supposent qu'une telle vitesse de rotation de l'agitateur mécanique inférieure à 300 rpm est insuffisante pour permettre la formation d'une émulsion entre l'acide oléique et l'oxydant (H₂O₂) et entraine la diminution du rendement.

### EXEMPLE 7 - Scission oxydative de l'acide 9,10-époxyoctadécanoïque.

On prépare de l'acide 9,10-époxyoctadécanoïque selon une méthode décrite dans « Salimon et al., (2009), European Journal of Scientific Research, 32(2), 216-222 » et dans laquelle on traite 71 mmoles d'acide oléique avec de l'acide performique fraichement préparé à température ambiante pendant 3 heures sous agitation magnétique (400 rpm). On obtient une poudre blanche d'acide 9,10-époxyoctadécanoïque (51 mmoles) avec un taux de pureté de 65 % et un rendement de 72 %.

On réalise un traitement de scission moléculaire oxydative de l'acide 9,10-époxyoctadécanoïque dans lequel on forme *in situ* le catalyseur (B) dans une proportion molaire de 2 %. La quantité d'acide 9,10-époxyoctadécanoïque initiale est de 51 mmoles et la quantité de H₂O₂ initiale est de 255 mmoles (30 %). Le rapport molaire 9,10-époxyoctadécanoïque/H₂O₂ initial est de 1/5, la température est de 85°C, la durée de réaction est de 5 heures et la vitesse de rotation de l'agitateur mécanique est de 400 rpm.

Le taux de conversion « TC » de l'acide 9,10-époxyoctadécanoïque, « AZA % » et « PEA % » représentant respectivement la valeur du rendement de synthèse de l'acide azélaïque et de l'acide pélargonique par rapport à l'acide 9,10-époxyoctadécanoïque de départ est donné au tableau 7 ci-après.

**Tableau 7**

| Catalyseur (B) préparé *in situ* | TC, % | AZA % | PEA % |
|---|---|---|---|
| (C₅H₅N(*n*-C₁₆H₃₃))₃{PO₄[WO(O₂)₂]₄} | 98,9 | 86,5 | 87,3 |

### EXEMPLE 8 - Scission oxydative de l'acide linoléique (LNA).

On réalise un traitement de scission moléculaire oxydative de l'acide linoléique (acide (9*cis*,12*cis*)-octadéca-9,12-diènoïque, LNA) selon les essais n°1 et n° 2 dans lesquels on forme le catalyseur (B) *in situ* dans l'acide linoléique (LNA). La proportion molaire LNA/H₂O₂/catalyseur est précisée au tableau 8 ci-après. La valeur « LNA % » représente la fraction molaire d'acide linoléique transformée lors de la réaction. Les valeurs « HA % » et « AZA % » représentent respectivement la valeur du rendement de synthèse et d'extraction du monoacide hexanoïque (HA) et du diacide azélaïque (AZA) par rapport à l'acide linoléique (LNA) de départ. La température est de 85°C, la durée de réaction est de 5 heures et la vitesse d'agitation est de 400 rpm.

**Tableau 8**

| | Essai n° 1 | Essai n° 1 |
|---|---|---|
| LNA, mmoles | 60 | 22 |
| H₂O₂, mmoles | 350 | 220 |
| LNA/H₂O₂ | 1/5,8 | 1/10 |
| Cat (B), mmoles | 1,2 | 0,9 |
| LNA/Cat (B) | 1/50 (0,02 %) | 1/25 (0,04 %) |
| LNA % | 100 | 100 |
| HA % | 47,7 | 60,7 |
| AZA % | 41,0 | 49,8 |

### EXEMPLE 9 - Scission oxydative de l'acide ricinoléique.

On réalise un traitement de scission moléculaire oxydative de l'acide ricinoléique (ou acide (9*cis*)-12-hydroxyoctadéca-9-ènoïque) issu de l'hydrolyse d'huile de ricin, tel que décrit à l'exemple 1 dans lequel on forme *in situ* le catalyseur (B) dans une proportion molaire à 2 %. La quantité initiale d'acide ricinoléique est de 14 mmol et la quantité de H₂O₂ initiale est de 163 mmol (30 %).

La température est de 85°C, la durée de réaction est de 5 heures et la vitesse d'agitation est de 400 rpm.

La scission moléculaire oxydative de l'acide ricinoléique conduit à la formation d'un diacide (acide azélaïque, AZA) et d'un mono-acide (acide 3-hydroxynonanoïque) qui sont extraits dans l'acétate d'éthyle.

On obtient l'acide azélaïque et l'acide 3-hydroxynonanoïque avec des rendements respectifs de 83,2 % et 60,8 % pour un taux de conversion de l'acide ricinoléique de 99,9 %.

## Revendications

1. Procédé de scission moléculaire oxydative d'un composé gras choisi dans le groupe formé des acides carboxyliques aliphatiques insaturés, des esters d'un acide carboxylique aliphatique insaturé, des acides carboxyliques aliphatiques époxydés, des esters d'un acide carboxylique aliphatique époxydé, des acides carboxyliques aliphatiques insaturés hydroxylés et des esters d'un acide carboxylique aliphatique insaturé hydroxylé, dans lequel :
- on forme une composition, dite composition grasse, constituée d'au moins un acide carboxylique aliphatique, ladite composition grasse comprenant le composé gras ;
**caractérisé en ce que** :
- on ajoute ensuite à ladite composition grasse une solution d'au moins un sel d'ammonium quaternaire dans l'eau apte à former une émulsion dudit composé gras et de l'eau, puis ;
- on ajoute à ladite émulsion une solution liquide d'au moins un acide tungsto-phosphorique dans une composition comprenant du peroxyde d'hydrogène (H₂O₂),
de façon à former *in situ* dans l'émulsion une quantité d'un catalyseur, dit catalyseur de transfert de phase, formé de l'acide tungsto-phosphorique et d'au moins un ammonium quaternaire du (des) sel(s) d'ammonium quaternaire(s) et à permettre la scission moléculaire oxydative du composé gras.

2. Procédé selon la revendication 1, **caractérisé en ce qu'**on choisit le composé gras dans le groupe formé des composés gras de formule générale (II) suivante :
R₁-(CH₂)ₐ-R₂-(CH₂)_{b}-R₃-(CH₂)_{c}-R₄-(CH₂)_{d}-R₅-(CH₂)ₑ-R₆ (II),
dans laquelle :
o R₁ et R₆ sont deux groupements d'atomes identiques ou distincts choisis parmi un méthyle (-CH₃) et un carboxyle (-COOH) et un groupement ester de formule générale -COOR₇ dans laquelle R₇ est un groupement hydrocarboné aliphatique linéaire comprenant de 1 à 8 atomes de carbone;
∘ R₂, R₃, R₄ et R₅ sont des groupements d'atomes identiques ou distincts choisis parmi un groupe -CH₂-, un groupe époxyde 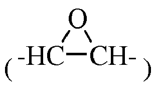 et un groupe (-CH=CH-) ;
o a, b, c, d et e sont des nombres entiers naturels identiques ou distincts de l'intervalle [0 ; 15].

3. Procédé selon l'une des revendications 1 ou 2, **caractérisé en ce qu'**on choisit chaque sel d'ammonium quaternaire dans le groupe formé des sels d'ammonium quaternaires de formule générale (III) suivante :
A₁A₂A₃A₄N⁺X⁻ (III) ;
dans laquelle :
∘ A₁, A_{2,} A₃ et A₄ sont des groupements hydrocarbonés aliphatiques identiques ou distincts présentant un nombre d'atomes de carbone inférieur à 10, et ;
o X⁻ est un anion.

4. Procédé selon l'une des revendications 1 à 3, **caractérisé en ce que** la composition grasse est exempte de solvant organique.

5. Procédé selon l'une des revendications 1 à 4, **caractérisé en ce que** le composé gras est l'acide oléique (acide (9*cis*)-octadéc-9-énoïque) et **en ce que** les produits formés sont l'acide azélaïque (HOOC-(CH₂)₇-COOH) et l'acide pélargonique (H₃C-(CH₂)₇-COOH).

6. Procédé selon l'une des revendications 1 à 4, **caractérisé en ce que** le composé gras est l'acide linoléique (acide (9*cis*,12*cis*)-octadéca-9,12-diènoïque) et **en ce que** les produits formés par scission moléculaire oxydative de l'acide linoléique sont l'acide azélaïque (HOOC-(CH₂)₇-COOH), l'acide malonique (HOOC-CH₂-COOH) et l'acide hexanoïque (H₃C-(CH₂)₄-COOH).

7. Procédé selon l'une des revendications 1 à 4, **caractérisé en ce que** le composé gras est l'acide 9,10-époxyoctadécanoïque et **en ce que** les produits formés par scission moléculaire oxydative de l'acide 9,10-époxyoctadécanoïque sont l'acide azélaïque (HOOC-(CH₂)₇-COOH) et l'acide pélargonique (H₃C-(CH₂)₇-COOH).

8. Procédé selon l'une des revendications 1 à 4, **caractérisé en ce que** le composé gras est l'acide arachidonique et **en ce que** les produits formés par scission moléculaire oxydative de l'acide arachidonique sont l'acide hexanoïque (H₃C-(CH₂)₄-COOH), l'acide malonique (HOOC-CH₂-COOH) et l'acide pentanedioïque (HOOC-(CH₂)₃-COOH).

9. Procédé selon l'une des revendications 1 à 4, **caractérisé en ce que** le composé gras est l'acide linolénique et **en ce que** les produits formés par scission moléculaire oxydative de l'acide linolénique sont l'acide azélaïque (HOOC-(CH₂)₇-COOH), l'acide propionique (H₃C-CH₂-COOH) et l'acide malonique (HOOC-CH₂-COOH).

10. Procédé selon l'une des revendications 1 à 9, **caractérisé en ce que** la proportion molaire des insaturations du composé gras et du peroxyde d'hydrogène introduits dans l'émulsion est comprise entre 1/4 et 1/10.

11. Procédé selon l'une des revendications 1 à 10, **caractérisé en ce que** la proportion molaire des insaturations du composé gras et de l'acide tungsto-phosphorique introduits dans l'émulsion est comprise entre 1 % et 5 %.

12. Procédé selon l'une des revendications 1 à 11, **caractérisé en ce que** l'acide tungsto-phosphorique et l'ammonium quaternaire introduits dans l'émulsion présentent une proportion équimolaire dans l'émulsion.

13. Procédé selon l'une des revendications 1 à 12, **caractérisé en ce qu'**on réalise la scission moléculaire oxydative du composé gras à une température comprise entre 60°C et 120°C.

14. Procédé selon l'une des revendications 1 à 13, **caractérisé en ce qu'**à l'issue de la scission moléculaire oxydative du composé gras, on récupère par filtration le catalyseur de transfert de phase et on utilise ledit catalyseur de transfert de phase dans une étape subséquente de scission moléculaire oxydative d'un second composé gras.

## Patentansprüche

1. Verfahren zur oxidativen molekularen Spaltung einer Fettverbindung, ausgewählt aus der Gruppe, bestehend aus ungesättigten aliphatischen Carbonsäuren, Estern einer ungesättigten aliphatischen Carbonsäure, epoxydierten aliphatischen Carbonsäuren, Estern einer epoxydierten aliphatischen Carbonsäure, hydroxylierten ungesättigten aliphatischen Carbonsäuren, und Estern einer hydroxylierten ungesättigten aliphatischen Carbonsäure, wobei
- eine Zusammensetzung, genannt Fettzusammensetzung, gebildet wird, die aus mindestens einer aliphatischen Carbonsäure besteht, wobei die Fettzusammensetzung die Fettverbindung umfasst;
**dadurch gekennzeichnet, dass**:
- dann der Fettzusammensetzung eine Lösung aus mindestens einem quartären Ammoniumsalz in Wasser zugegeben wird, die ausgelegt ist, um eine Emulsion der Fettverbindung und des Wassers zu bilden, dann
der Emulsion eine flüssige Lösung aus mindestens einer Tungsto-Phosphorsäure in einer Zusammensetzung zugegeben wird, die Wasserstoffperoxid (H₂O₂) umfasst,
um *in situ* in der Emulsion eine Menge eines Katalysators, genannt Phasentransferkatalysator, zu bilden, der aus Tungsto-Phosphorsäure und mindestens einem quartären Ammonium des/der quaternären Ammoniumsalze (s) gebildet ist, und eine oxidative molekulare Spaltung der Fettverbindung zu ermöglichen.

2. Verfahren nach Anspruch 1, **dadurch gekennzeichnet, dass** die Fettverbindung aus der Gruppe gewählt wird, gebildet aus Fettverbindungen mit der folgenden allgemeinen Formel (II):
R₁-(CH₂)ₐ-R₂-(CH₂)_{b}-R₃-(CH₂)_{c}-R₄-(CH₂)_{d}-R₅-(CH₂)ₑ-R₆ (II),
wobei:
- R₁ und R₆ zwei identische oder verschiedene Gruppen von Atomen sind, ausgewählt aus einem Methyl (-CH₃) und einem Carboxyl (-COOH) und einer Estergruppe mit der allgemeinen Formel -COOR₇, wobei R₇ eine lineare aliphatische Kohlenwasserstoffgruppe mit 1 bis 8 Kohlenstoffatomen ist;
- R₂, R₃, R₄ und R₅ Gruppen von identischen oder verschiedenen Atomen sind, ausgewählt aus einer -CH₂- Gruppe, einer Epoxidgruppe 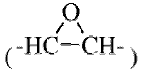 und einer (-CH=CH-)-Gruppe;
- a, b, c, d und e identische oder verschiedene natürliche ganze Zahlen des Intervalls [0; 15] sind.

3. Verfahren nach einem der Ansprüche 1 oder 2, **dadurch gekennzeichnet, dass** jedes quartäre Ammoniumsalz aus der Gruppe ausgewählt wird, gebildet aus quaternären Ammoniumsalzen mit der Folgenden allgemeinen Formel (III):
A₁A₂A₃A₄N⁺X⁻ (III);
wobei:
- A₁, A₂, A₃ und A₄ identische oder verschiedene aliphatische Kohlenwasserstoffgruppen sind, die eine Anzahl von Kohlenstoffatomen aufweisen, die geringer als 10 ist; und
- X⁻ ein Anion ist.

4. Verfahren nach einem der Ansprüche 1 bis 3, **dadurch gekennzeichnet, dass** die Fettzusammensetzung frei von organischem Lösemittel ist.

5. Verfahren nach einem der Ansprüche 1 bis 4, **dadurch gekennzeichnet, dass** die Fettverbindung Ölsäure ist ((9_{cis})-Octadec-9-ensäure) und dadurch, dass die gebildeten Produkte Azelainsäure (HOOC-(CH₂)₇-COOH) und Pelargonsäure (H₃C-(CH₂)₇-COOH) sind.

6. Verfahren nach einem der Ansprüche 1 bis 4, **dadurch gekennzeichnet, dass** die Fettverbindung Linolsäure ((9*cis*, 12*cis*)-octadeca-9,12-diensäure) ist, und dadurch, dass die Produkte, gebildet durch oxidative molekulare Spaltung der Linolsäure, Azelainsäure (HOOC-(CH₂)₇-COOH), Malonsäure (HOOC-CH₂-COOH) und Hexansäure (H₃C-(CH₂)₄-COOH) sind.

7. Verfahren nach einem der Ansprüche 1 bis 4, **dadurch gekennzeichnet, dass** die Fettverbindung 9,10-Epoxyoctadecansäure ist, und dadurch, dass die Produkte, gebildet durch oxidative molekulare Spaltung der 9,10-Epoxyoctadecansäure, Azelainsäure (HOOC-(CH₂)₇-COOH) und Pelargonsäure (H₃C-(CH₂)7-COOH) sind.

8. Verfahren nach einem der Ansprüche 1 bis 4, **dadurch gekennzeichnet, dass** die Fettverbindung Arachidonsäure ist, und dadurch, dass die Produkte, gebildet durch oxidative molekulare Spaltung der Arachidonsäure, Hexansäure (H₃C-(CH₂)₄-COOH), Malonsäure (HOOC-CH₂-COOH) und Pentandisäure (HOOC- (CH₂)₃-COOH) sind.

9. Verfahren nach einem der Ansprüche 1 bis 4, **dadurch gekennzeichnet, dass** die Fettverbindung Linolsäure ist, und dadurch, dass die Produkte, gebildet durch oxidative molekulare Spaltung der Linolsäure, Azelainsäure (HOOC-(CH₂)₇-COOH), Propionsäure (H₃C-CH₂-COOH) und Malonsäure (HOOC-CH₂-COOH) sind.

10. Verfahren nach einem der Ansprüche 1 bis 9, **dadurch gekennzeichnet, dass** die molare Proportion der Unsättigungen der Fettverbindung und des Wasserstoffperoxids, die in die Emulsion eingeführt sind, zwischen 1/4 und 1/10 liegt.

11. Verfahren nach einem der Ansprüche 1 bis 10, **dadurch gekennzeichnet, dass** die molare Proportion der Unsättigungen der Fettverbindung und der Tungsto-Phosphorsäure, die in die Emulsion eingeführt sind, zwischen 1 % und 5 % liegt.

12. Verfahren nach einem der Ansprüche 1 bis 11, **dadurch gekennzeichnet, dass** die Tungsto-Phosphorsäure und das quaternäre Ammonium, die in die Emulsion eingeführt sind, eine äquimolare Proportion in der Emulsion aufweisen.

13. Verfahren nach einem der Ansprüche 1 bis 12, **dadurch gekennzeichnet, dass** die oxidative molekulare Spaltung der Fettverbindung bei einer Temperatur durchgeführt wird, die zwischen 60 °C und 120 °C liegt.

14. Verfahren nach einem der Ansprüche 1 bis 13, **dadurch gekennzeichnet, dass** nach der oxidativen molekularen Spaltung der Fettverbindung durch Filtrierung der Phasentransferkatalysator wiedergewonnen wird und der Phasentransferkatalysator in einem nachfolgenden Schritt des oxidativen molekularen Spaltens einer zweiten Fettverbindung verwendet wird.

## Claims

1. A method of oxidative molecular cleavage of a fatty compound selected from the group formed of unsaturated aliphatic carboxylic acids, esters of an unsaturated aliphatic carboxylic acid, epoxidized aliphatic carboxylic acids, esters of an epoxidized aliphatic carboxylic acid, hydroxylated unsaturated aliphatic carboxylic acids, and esters of a hydroxylated unsaturated aliphatic carboxylic acid, wherein:
- a composition, named a fatty composition, composed of at least one aliphatic carboxylic acid is formed, said fatty composition comprising the fatty compound; **characterized in that**:
- subsequently adding to said fatty composition a solution of at least one quaternary ammonium salt in water capable of forming an emulsion of said fatty compound and water; and then
- adding to said emulsion a liquid solution of at least one tungstophosphoric acid in a composition comprising hydrogen peroxide (H₂O₂),
so as to form *in situ* in the emulsion a quantity of a catalyst, named a phase-transfer catalyst, formed of tungstophosphoric acid and at least one quaternary ammonium of the quaternary ammonium salt(s), and to permit the oxidative molecular cleavage of the fatty compound.

2. Method according to claim 1, **characterized in that** the fatty compound is selected from the group formed of the fatty compounds of the following general formula (II):
R₁(CH₂)ₐ-R₂-(CH₂)_{b}-R₃-(CH₂)_{c}-R₄-(CH₂)_{d}-R₅-(CH₂)ₑ-R₆ (II),
wherein:
∘ R₁ and R₆ are two identical or different groups of atoms selected from a methyl (-CH₃) and a carboxyl (-COOH) and an ester group of the general formula -COOR₇ wherein R₇ is a linear aliphatic hydrocarbon group containing from 1 to 8 carbon atoms;
∘ R₂, R₃, R₄ and R₅ are identical or different groups of atoms selected from a group -CH₂-, an epoxide group 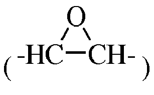 and a group (-CH=CH-);
o a, b, c, d and e are identical or different natural integers of the interval [0 ; 15].

3. Method according to one of claims 1 or 2, **characterized in that** each quaternary ammonium salt is selected from the group formed of the quaternary ammonium salts of the following general formula (III):
A₁A₂A₃A₄N⁺X⁻ (III),
wherein:
∘ A₁, A₂, A₃ and A₄ are identical or different aliphatic hydrocarbon groups having a number of carbon atoms of less than 10; and
∘ X⁻ is an anion.

4. Method according to any one of claims 1 to 3, **characterized in that** the fatty composition is free of organic solvent.

5. Method according to any one of claims 1 to 4, **characterized in that** the fatty compound is oleic acid ((9*cis*)-octadec-9-enoic acid) and **in that** the products formed are azelaic acid (HOOC-(CH₂)₇-COOH) and pelargonic acid (H₃C-(CH₂)₇-COOH).

6. Method according to any one of claims 1 to 4, **characterized in that** the fatty compound is linoleic acid ((9*cis,* 12*cis*)-octadeca-9,12-dienoic acid) and **in that** the products formed by oxidative molecular cleavage of the linoleic acid are azelaic acid (HOOC-(CH₂)₇-COOH), malonic acid (HOOC-CH₂-COOH) and hexanoic acid (H₃C-(CH₂)₄-COOH).

7. Method according to any one of claims 1 to 4, **characterized in that** the fatty compound is 9,10-epoxyoctadecanoic acid and **in that** the products formed by oxidative molecular cleavage of the 9,10-epoxyoctadecanoic acid are azelaic acid (HOOC-(CH₂)₇-COOH) and pelargonic acid (H₃C-(CH₂)₇-COOH).

8. The method according to any one of claims 1 to 4, **characterized in that** the fatty compound is arachidonic acid and **in that** the products formed by oxidative molecular cleavage of the arachidonic acid are hexanoic acid (H₃C-(CH₂)₄-COOH), malonic acid (HOOC-CH₂-COOH) and pentanedioic acid (HOOC-(CH₂)₃-COOH).

9. Method according to any one of claims 1 to 4, **characterized in that** the fatty compound is linolenic acid and **in that** the products formed by oxidative molecular cleavage of the linolenic acid are azelaic acid (HOOC-(CH₂)₇-COOH), propionic acid (H₃C-CH₂-COOH) and malonic acid (HOOC-CH₂-COOH).

10. Method according to any one of claims 1 to 9, **characterized in that** the molar proportion of the unsaturated bonds of the fatty compound and of the hydrogen peroxide introduced into the emulsion is from 1/4 to 1/10.

11. Method according to any one of claims 1 to 10, **characterized in that** the molar proportion of the unsaturated bonds of the fatty compound and of the tungstophosphoric acid introduced into the emulsion is from 1% to 5%.

12. Method according to any one of claims 1 to 11, **characterized in that** the tungstophosphoric acid and the quaternary ammonium introduced into the emulsion have an equimolar proportion in the emulsion.

13. Method according to any one of claims 1 to 12, **characterized in that** the oxidative molecular cleavage of the fatty compound is carried out at a temperature of from 60°C to 120°C.

14. Method according to any one of claims 1 to 13, **characterized in that**, at the end of the oxidative molecular cleavage of the fatty compound, the phase-transfer catalyst is recovered by filtration and said phase-transfer catalyst is used in a subsequent step of oxidative molecular cleavage of a second fatty compound.
